Europäisches Patentamt

(19) European Patent Office

Office européen des brevets

(11) Publication number: **0 266 331**
**A2**

(12) # EUROPEAN PATENT APPLICATION

(21) Application number: 87850324.2

(22) Date of filing: 28.10.87

(51) Int. Cl.⁴: **G 01 N 33/48**
**G 01 N 33/66**

(30) Priority: **30.10.86 US 924759**

(43) Date of publication of application:
**04.05.88 Bulletin 88/18**

(84) Designated Contracting States:
**AT BE CH DE ES FR GB GR IT LI LU NL SE**

(71) Applicant: **LEJUS MEDICAL AKTIEBOLAG**
**Taljegardsgatan 3**
**S-431 33 Mölndal (SE)**

(72) Inventor: **Smith, Ulf Per Gustav**
**Strandrägsvägen 8**
**S-434 00 Kungsbacka (SE)**

**Lönnroth, Nils Peter**
**Korpäsvägen 3**
**S-433 76 Partille (SE)**

(74) Representative: **Inger, Lars Ulf Bosson**
**L + U INGER Patentbyra AB Garvaregatan 12**
**S-262 00 Ängelholm (SE)**

(54) Method for the determination of the concentration of substances in intercellular tissue fluids.

(57) The present invention relates to a method for the determination of concentration of substances in intercellular tissue fluid, whereby the method comprises
   i) placing a dialysis fibre tube in a tissue, whereby the dialysis fibre membrane has a cut-off for the molecular weight determined by the substance to be measured;
   ii) perfusing an isotonic saline solution through said fibre;
   iii) analysing the dialysate with regard to qualitative and quantitative parameters.

Bundesdruckerei Berlin

**Description**

## A METHOD FOR THE DETERMINATION OF THE CONCENTRATION OF SUBSTANCES IN INTERCELLULAR TISSUE FLUID.

The present invention relates to a new method for the determination of the concentration of different substances in intercellular tissue fluid.

The object of the present invention is to provide a possibility to determine with high accuracy the concentration as well as the presence of substances in the intercellular tissue fluid. Another objective is thereby to provide a possibility to determine the distribution of substances around the cells of a tissue, when administering such substances to the tissue. A further objective is thereby to obtain a possibility to assay the distribution of pharmaceutically active compounds and their metabolites in a tissue, as well as diagnosing the presence of disease related substances in a tissue.

Background of the invention

When studying the distribution and effectiveness of pharmaceutically active compounds today their concentration, and presence in the blood serum is primarily determined. The determinations made do, however, only reflect the situation in the blood serum and the actual situation around the cells where the actual effect of e.g. a drug takes place is not shown at all. Besides the fact that onset times at cell level are badly reflected, the actual duration and metabolic situation in the cell is not shown at all. The blood serum assay is thus a primitive and coarse method, but still today the only one actually relied upon.

There is thus a scientific as well as a researching demand to obtain a more accurate method of determining the concentration of substances in intercellular tissue fluid thereby to get a better tool e.g. for the understanding of effects effectuated in the cells. This would as well improve e.g. the tools for allowing/nonallowing pharmaceutically active compounds.

Description of the present invention

It has now surprisingly been shown possible to solve these problems and meet the demand set forth above by means of the present invention which is characterized in

i) placing a dialysis fibre tube in a tissue, whereby the dialysis fibre membrane has a cut-off for the molecular weight determined by the substance to be measured;

ii) perfusing an isotonic saline solution through said fibre tube,

iii) analysing the dialysate with regard to qualitative and quantitative parameters.

The recovery factor in vivo of the dialysis fibre is determined by perfusing an isotonic saline solution containing different concentrations of the substance to be measured.

In a preferred embodiment of the invention a substance to be assayed is administered by the isotonic saline solution and the difference between ingoing concentration of the substance in the perfusate and the concentration of the substance in the dialysate is determined.

The invention will be described below with reference to a series of tests carried out on glucose.

A small tube of dialysis fibre having a cut-off value for the molecular weight related to the substance to be determined is implanted into a tissue and perfused with an isotonic saline solution. The difference between the concentration of the substance looked for in the dialysis fibre and in the tissue fluid is a gradient over the membrane of the dialysis fibre. A certain amount of this difference will be brought into the solution in the dialysis fibre and is thus a measure of the recovery of the fibre of the substance in question.

The recovery at the dialysis concentration in dialysate/concentration in medium of a given substance can be determined in vitro but will not correspond with the recovery in vivo.

Thus one can not calculate the concentration of different substances in the tissue liquid with reference to the recovery of the dialysis fibre in vitro as such.

If on the other hand the dialysis fibre is perfused with a number of known concentrations of the substance looked for, one can, having the knowledge that the recovery is constant in all gradient sizes, lay down the following relation between the recovery of the substance in question and the known concentrations perfused:

$$f(x-a_1) = b_1-a_1 = \Delta_1 \quad (1)$$
$$f(x-a_2) = b_2-a_2 = \Delta_2 \quad (2)$$
$$f(x-a_x) = 0 \quad (3)$$

f is the recovery factor

x is the concentration sought in the tissue liquid

$a_{1-x}$ is the known concentration in the fibre prior to dialysis

$b_{1-x}$ is the concentration in the fibre after dialysis

$\Delta_{1-x}$ is the net increase of the concentration in the fibre after dialysis.

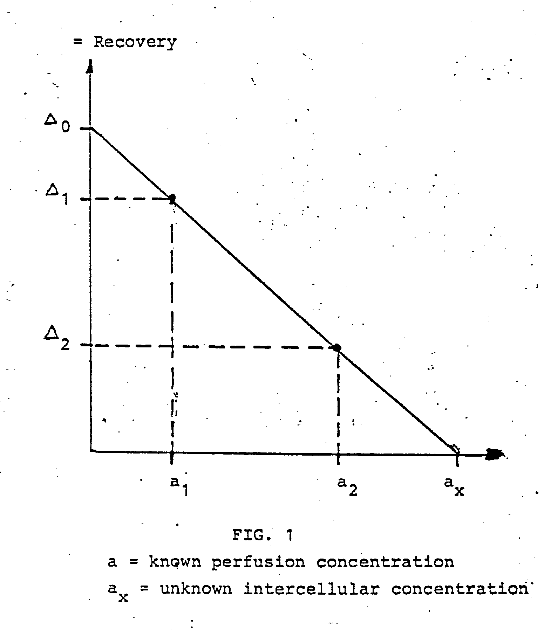

The different factors describes a linear relationship which is illustrated in FIG. 1. It is evident from the figure that one can extrapolate the concentration ($a_x$) in the tissue of the substance looked for with reference to the results from some measuring points.

More specifically the dialysis fibre is connected to a perfusion tube and this is connected to a micropump.

The dialysis fibre part is implanted in the tissue. The micropump perfuses the dialysis aggregate with an isotonic saline solution, samples for analysis are taken from the dialysate, i.e., the effluent solution from the dialysis fibre.

The concentration of the substance sought for in the dialysate is analyzed and with reference to the known concentration of said substance in the perfusate prior to dialysis the linear function can be established as per above and the equation of the line is obtained by regression analysis.

In an experimental situation the equation of the line is thus:

$$\Delta_{1-x} = \Delta_0 - fa_{1-x} \quad (4)$$

$\Delta_0$ is the net increase of the concentration of the substance given in the dialysis fibre after a dialysis using isotonic saline solution without any addition at all of the substance given. The other symbols are analogous with the prior equations (1-3).

When $\Delta = 0$ then is $a = x$, i.e. $a_x$ is x.

The following tests have been carried out using glucose as a model substance. The concentration of glucose in a tissue has been related to the concentration of glucose obtained from venous blood, as these concentrations at a steady state situation shall and will correspond well with each other.

A sterilized, 30 mm long dialysis fibre having an outer diameter of 0.30 mm (type Cuprophan Hollow Fibre) having a cut-off for the molecular weight related to the substance given, in this case a cut-off of 3000, is introduced through a part of the abdominal subcutaneous tissue of a human, and is connected to a micropump (SAGE). The dialysis aggregate is perfused with isotonic saline solution comprising known concentrations of glucose, using a pump rate of 2.5 µl/min.

A sample of the dialysate is taken for the analysis of glucose every six minute.

The amount of glucose is determined using the glucoseoxidase method. In accordance with the above given formulae (1-4) the recovery factor, the unknown tissue concentration, and the blood sugar concentration are determined. Further a determination of the recovery factor of the same fibre bathed in an isotonic saline solution comprising a known concentration of glucose is made.

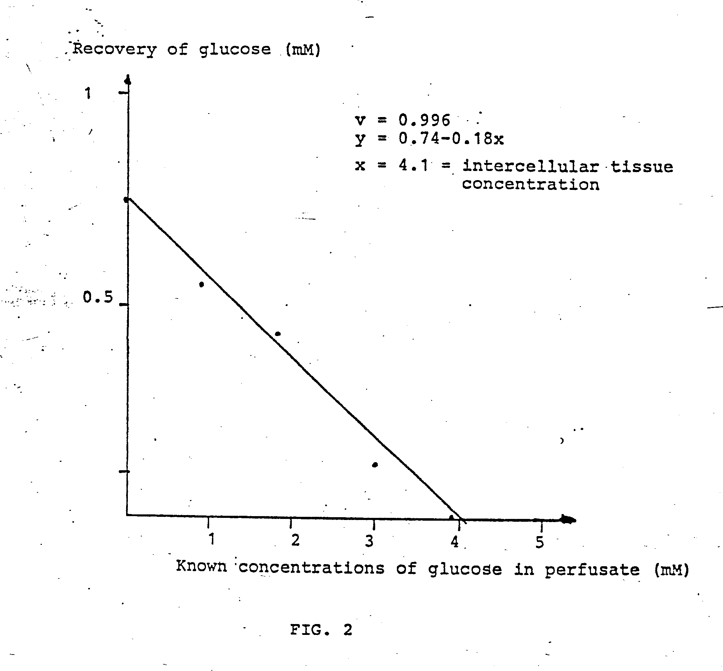

The results of typical analysis during steady state conditions are given in FIG. 2. The correlation coefficients of the lines are 0.996, which means a high statistic significanse. This also implicates that stable steady state conditions (a constant recovery) was kept for at least 4 hrs. Calculated concentrations of glucose in the tissue liquid are 4.1 mM to be compared with a blood sugar concentration of 4.0 mM. The inclination coefficient, i.e. the recovery factor is 0.18 to be compared with the recovery factor of the fibre and obtained in an isotonic saline solution comprising a known concentration of glucose, which factor then is 0.44. Thus the recovery factor in vivo is completely different from the one found in vitro.

From 22 different measurements made on humans a mean recovery factor of 0.2±60.02 (mean value ±SEM) was obtained.

The mean tissue concentration was simultaneously 4.7±0.3 mM to be compared with the blood sugar concentrations during the same measurements which was 4.7±0.3 mM. If two dialysis fibres are implanted and four calibration measurement are obtained the mean divergence will be 6±2%. By knowledge of the mean recovery for glucose it was also possible to measure tissue levels of glucose in short term experiments, where the dialysis probe was perfused with only isotonic saline. In this type of measurement the mean relative difference between estimated tissue glucose and the concentration in venous blood was 12±2% (n=14).

When the tissue concentration has been obtained at steady state a stress test is made. In FIG. 3 the results of this test are given, whereby the tissue concentration of glucose is compared with the blood sugar concentration after the patient has been drinking an aqueou solution containing 100 g of glucose. One can clearly see that there is a time difference between the tissue concentration graph and the blood sugar concentration graph, which means that the regional tissue concentration of glucose has really been measured using the dialysis fibre, and that the kinetics of glucose can be different in the tissue compared with venous blood.

In the test shown above the dialysis fibre was implanted in abdominal subcutaneous tissue, but it is evident that the fibre can be implanted into any tissue, and that different fibres can be implanted in different tissues simultaneously to register the events of an administration of an active substance.

The cut-off for the molecular weight of the fibre was 3000 in the example shown above, but this is then of course dependent on the substance to be found. When more heavy compounds are to be tested, such as peptides, and hormones, a cut-off value of 10000-20000 is used. Thus the cut-off value can be varied considerably.

In a further test on 5 healthy volounters the dialysis fibres were placed in subcutaneous tissue. The patients had taken per-orally 80 mg of propranolol 10 hrs before testing. The results of the tests were:

| | Plasma conc. | Tissue conc. | % |
|---|---|---|---|
| Mv | 26 nM | 6 nM | 23 |

At a renewd administration of 80 mg of propranolol no significant increase of the propranolol concentration

3

in the fat tissue can be observed during the observation period of 50-120 min. During this time period the plasma concentration increases from 26 nM to a maximum of 320 nM at the time of the peak, about 60 min after administration.

Thus there can be seen a considerably lower tissue concentration than plasma concentration. Further, quite another kinetics can be observed in the subcutaneous tissue compared with the plasma.

In another test on pig dialysis fibres were implanted in the heart as well as in subcutaneous tissue. Propranolol was infundated from 0 min to 40 min at a rate of 0.5 mg/h, after an initial priming dose of 1 mg given intravenously. The results are given in the Table below.

TABLE.

| Time Target | 5 | 10 | 20 | 30 | 40 | 60 | 70 | 80 | 100 | 120 min |
|---|---|---|---|---|---|---|---|---|---|---|
| Plasma nM | 180 | 167 | 184 | 176 | 92 | 59 | 43 | 34 | 26 | 15 |
| Heart nM | 0.3 | 0.5 | 0.5 | 0.9 | 0.7 | 0 | 0 | 0 | 0 | 0 |
| Fat tissue nM | 0 | 0 | 0 | 0 | 0 | 0 | 0 | 0 | 0 | 0 |

Thus there can be seen that the peak concentration in the heart is about 0.5% of the peak plasma concentration; that the kinetics differs but is more similar to that of the plasma than that of the fat tissue; and that there is no detectable amounts of propranolol in fat tissue.

## Claims

1. An apparatus for the determination of concentration of substances in intercellular tissue fluid, characterized in an dialysis fibre tube arranged to be placed in a tissue, whereby the dialysis fibre membrane has a cut-off for the molecular weight determined by the substance to be measured; a device for perfusing an isotonic saline solution through said fibre; and means for collecting samples from a dialysate formed during perfusion.

2. An apparatus according to claim 1, wherein the cut-off for the molecular weight is 1000-3000, when to determine smaller substances.

3. An apparatus according to claim 1, wherein the cut-off for the molecular weight is above 10000, when to determine heavy substances.

FIG. 1

a = known perfusion concentration

$a_x$ = unknown intercellular concentration

Recovery of glucose (mM)

v = 0.996
y = 0.74-0.18x

x = 4.1 = intercellular tissue
concentration

Known concentrations of glucose in perfusate (mM)

FIG. 2